# EUROPEAN PATENT SPECIFICATION

(11) **EP 0 815 891 B1**
(45) Date of publication and mention of the grant of the patent: **28.04.2004**
(21) Application number: 97304356.5
(22) Date of filing: 20.06.1997
(51) Int. Cl.: A61M 16/16, A61M 16/00

(54) **Arrangement for processing respiratory gas of an intubated patient**
Einrichtung zur Behandlung von Atemgas eines intubierten Patienten
dispositif de traitement du gaz respiratoire d'un patient intubé

(30) Priority: 27.06.1996 FI 962665
(43) Date of publication of application: 07.01.1998
(73) Proprietor: Instrumentarium Corporation, 00510 Helsinki (FI)
(72) Inventor: Meriläinen, Pekka, 00660 Helsinki (FI)
(74) Representative: Charlton, Peter John

(56) References cited:
- EP-A- 0 243 259
- US-A- 5 172 686
- US-A- 5 349 946
- US-A- 5 429 123

## Description

The invention relates to an apparatus for processing respiratory gas of an intubated patient, wherein the humidity level of inhalation gas is raised, if necessary, before the gas is supplied to the patient.

Respiratory gas of a patient connected to a respirator must be humidified and heated artificially since the artificial respiratory passage obtained through the insertion of a respiratory tube, i.e. through intubation, passes the mucous membranes of the nasal cavity where the respiratory gas becomes moist and warm during natural respiration. Dry and cold respiratory gas irritates the trachea and the bronchi and cools down the patient's body temperature.

Two principal methods have been used previously to artificially humidify respiratory gas of a patient.

The first known method is the use of active humidifiers. Active humidifiers vaporise water into the air to be inhaled from a heated container connected to the pipes on the inhalation side.

The second known method is the use of an artificial nose, i.e. a humidity and moisture exchanger (HME). The artificial nose is connected directly to the end of the respiratory tube where it recovers humidity and heat from the exhaled air and stores them for release to the next inhalation phase.

Both of the aforementioned methods are problematic. Adjustment of a simple active humidifier is based on the heating capacity and on the visual monitoring of the amount of the humidity produced in the patient's tubes. Usually the humidification is easily excessive, so that a harmful amount of water may gather in the tubes. In the aforementioned situation, the water must be removed from the tubes at regular intervals. Another problem is that the circumstances in the container of the humidifier provide an advantageous environment for the growth of bacteria. An artificial nose, in turn, can never be used to humidify excessively since only 80 to 90% of the humidity contained in the exhaled air can be recovered and utilized in the next inhalation phase. Especially patients who are in intensive case and whose secretion of mucus is heavy require additional humidity to prevent the mucus gathering in the respiratory tube from drying up. The capacity of the artificial noses is not sufficient for this. The capacity of heat recovery of the artificial noses is not always sufficient, either.

US-A-5349946 discloses a humidification system humidifies breathable gas that is supplied to a patient with a ventilator or an anaesthesia circuit having inhalation tubing connecting from the ventilator to the patient and exhalation tubing extending from the patient. The humidification system includes a blotting paper humidifier disposed in the inhalation tubing adjacent to the patient Piping carries an amount of water from a reservoir to the humidifier, and the amount of water delivered to the humidifier is limited by a controllable pulsing valve disposed in the piping. A measurement device measures at least two parameters, and a regulation device regulates the pulsing valve as a function of the measured parameters so that an appropriate amount of water is delivered to the blotting paper to achieve a desired humidity level in the gas.

The purpose of the invention is to provide an apparatus with which the prior art drawbacks can be eliminated. This is achieved with the invention. The arrangement according to the invention is in turn characterized in that the arrangement further comprises a flow sensor arranged to carry out a measurement on the patient's respiratory air flow, second means arranged to calculate the respiratory volume on the basis of the performed measurement, and a control unit arranged to determine the amount of water discharged from the patient on the basis of the measured respiratory volume, and an actuator and a nozzle piece that are arranged, according to directions from the control unit, to supply an amount of water, dependent on the amount of water discharged from the patient, to the patient's respiratory tract during the next respiratory phase(s).

The advantage of the invention is that the prior art drawbacks can be eliminated effectively. The invention enables adjusting the amount of the humidity and the timing of its administration to accurately suit the needs of each patient, so that the overall result is very advantageous.

The invention will be described in greater detail below by means of a preferred embodiment described in the accompanying drawings, in which
Figure 1 is a general view of a system utilizing the method according to the invention,
Figure 2 is a general view of a possible spraying arrangement during the suction,
Figure 3 is a general view of the spraying arrangement of Figure 2 during the spraying, and
Figure 4 shows an alternative embodiment of a detail of Figure 1.

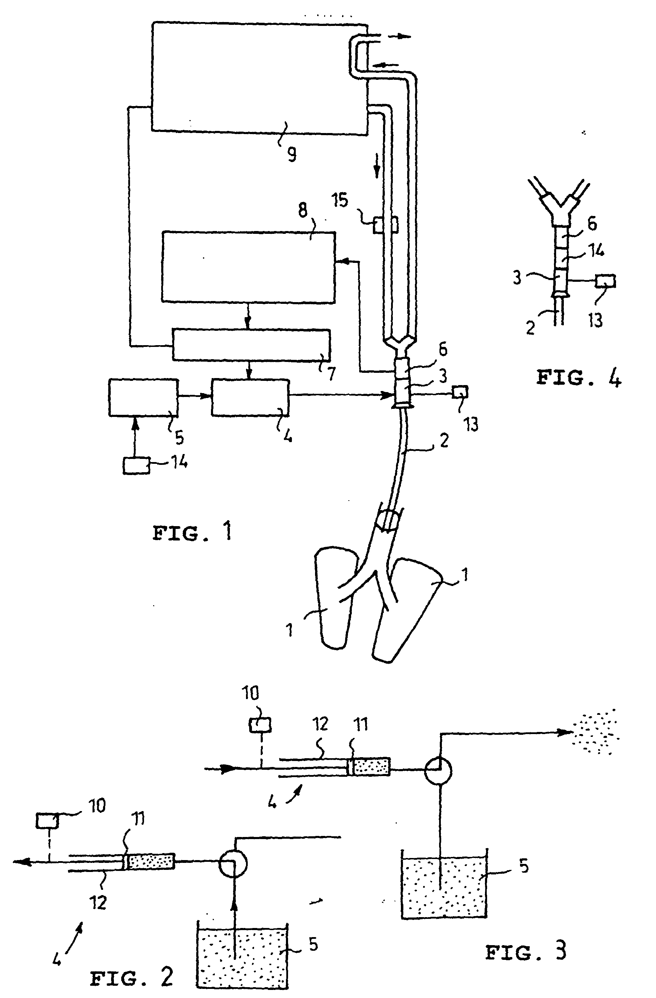

Figure 1 shows the characteristic features of a system utilizing the method according to the invention. Reference numeral 1 denotes a patient's lungs and reference numeral 2 a respiratory tube. Reference numeral 3 denotes a nozzle piece, numeral 4 an actuator and numeral 5 a water container. Reference numeral 6 denotes in Figure 1 a flow sensor and reference numeral 7 a control unit. Reference numeral 8 denotes in Figure 1 a flow monitor and reference numeral 9 a respirator.

According to the basic idea of the invention, a patient's respiratory volume, preferably the exhalation volume, is measured for example in the form of single respiratory volume or minute volume, the amount of water discharged from the patient is determined on the basis of the single respiratory volume measured from the exhalation, and a desired amount of water, dependent on the amount of water discharged from the patient, is supplied to the patient's respiratory tract for example through spraying during the next respiratory phase(s). The amount of water that is dependent on the amount of water discharged from the patient may be, for example, proportional to the amount of water discharged from the patient. The starting point is adjusting the amount of humidity and the timing of its administration to accurately suit the needs of the patient by spraying an exactly determined and timed dose of water in the form of spray or vapour to the patient's air passage during each inhalation phase, for example. The amount to be sprayed is measured by means of the flow sensor 6, and the flow monitor 8 and the control unit 7 connected thereto, in which case the flow sensor 6 carries out a measurement on the patient's respiratory air flow, the flow monitor 8 calculates the respiratory volume on the basis of the measurement, and the control unit 7 determines the amount of water that has been discharged from the patient according to the respiratory volume measured. An amount of water, dependent on the amount of discharged water determined in the aforementioned manner, is supplied to the patient for example through spraying during the next respiratory phase(s).

The sensor 6 may be, for example, a spirometric sensor described in US Patent 5,088,332 or in the corresponding Finnish Patent 84,757, used to measure, on the basis of a pressure difference, the single respiratory volume of the preceding exhalation period. The amount of water that has been removed from the patient can then be determined accurately by assuming that the gas arriving from the patient's lungs 1 is entirely saturated with water vapour. The amount of water discharged with a breath having a volume of one litre is typically about 30 mg or 30 mm³ when the temperature of the patient's exhalation is about 34°C. If required, measurement of the temperature of the exhalation air can be arranged to be carried out preferably from the end of the intubation tube. With the timing of the spraying at a certain stage of the inhalation it is also possible to control the proportion of the humidity reaching the pulmonary alveoli and the proportion remaining in the upper air passages. In this manner it is possible not only to replace the amount of water that has been discharged but to also increase selectively the humidity in the intubation tube 2 and the trachea, if necessary, to prevent the mucus from drying. The timing signal for the spraying can be obtained for example from the flow sensor 6. Another possibility is to obtain the timing signal from a ventilator or a respirator 9. The duration of the water supply process, preferably the spraying process, and its timing during the respiration, usually at the beginning of an inhalation or exhalation phase, is determined according to the needs in each situation.

The spraying of water or water vapour to the patient's respiratory circuit can be implemented in several manners. However, there are certain conditions related to the spraying that should be taken into account when designing a preferred embodiment. In order to prevent condensation, water must be sprayed as close to the orifice of the intubation tube 2 as possible and therefore to the patient side of the flow sensor 6. This also helps avoid the harmful effect of a possible aerosol or vapour spray to the flow measurement. The spraying device, pump or the like that acts as the actuator 4 must be able to supply a desired amount of water despite the high counterpressure, produced by the respirator, that prevails in the patient circuit and that can be as high as 60 to 80 cmH₂O. The compliance of the water pipe leading from the spraying device to the patient circuit must be very small so that even a small batch volume of water can be discharged from the nozzle piece 3 having a small diameter so that the water does not remain for example in the flexible volume of the pipe.

Figures 2 and 3 show an example of a possible spraying arrangement. The arrangement comprises a water container 5 and a piston 11, positioned in a cylinder 12, that forms the actuator 4 and that is controlled by a step motor 10. The piston 11 controlled by the step motor 10 sucks an amount of water that is dependent on the single respiratory volume of the exhalation to the cylinder 12 as the patient exhales, and empties the water to the respiratory tract next time the patient inhales. The emptying may take place either at the beginning of the inhalation in which case all the humidity reaches the lungs 1, or at the end in which case it remains in an anatomical dead space in the upper respiratory tract and in the intubation tube 2. Since the required volume of water may be very small in order that the dosage can be kept accurate, it is probably more advantageous to use a long and thin cylinder that has a small volume and that is filled and emptied during each breath, and not a large cylinder that would be filled less often.

The respiratory air can be heated either by heating the nozzle piece 3, in which case the same devices also act as water heaters, or by means of a separate heater 15. The nozzle piece 3 may be, for example, a short metal tube that is electrically heatable and that contains in its wall a small opening through which the water is sprayed. The devices used for heating the nozzle piece 3 are denoted in the figure by means of reference numeral 13.

The water can be supplied to the air passage either in the form of aerosol or gas. Both alternatives are technically easy to arrange and it might be even clinically useful that both forms could be produced with the same apparatus. Aerosol can be especially preferable if the apparatus is also to be used to administer pharmaceuticals, such as water-soluble drugs, to the patient's respiratory system. It should be mentioned that this is normally carried out with an ultrasonic apparatus or an ejector-type apparatus that uses oxygen as the operating gas. The drawback with a gas-operated nebulizer is that the oxygen level supplied to the patient changes with the installation of the apparatus and the measurement of the patient's oxygen consumption is also seriously disturbed. A nebulizer implemented according to the principle of the present invention does not have the aforementioned drawbacks. The supply of a pharmaceutical to the water to be sprayed is shown generally in Figure 1 by means of reference numeral 14.

Figure 4 shows a general view of an alternative embodiment of the arrangement of Figure 1. Like reference numerals refer to corresponding parts in Figure 4 and in Figure 1. In the embodiment shown in Figure 4, between the nozzle piece 3 and the flow sensor 6 there is a component 14 comprising an artificial nose (HME) or a bacterial filter or both.

The embodiment described above is in no way intended to restrict the invention, but the invention can be modified freely within the scope of the claims. Therefore it is clear that an actuator or other details in an apparatus utilizing the invention do not have to be exactly as shown in the figures, but other kinds of arrangements are also possible.

## Claims

1. An arrangement for processing respiratory gas of an intubated patient, the arrangement comprising means (2, 9) for supplying inhalation gas to the patient's lungs (1) and for conducting the exhalation gas from the patient, and humidifying means (5) for increasing the level of humidity in the inhalation gas, **characterized in that** the arrangement further comprises a flow sensor (6) arranged to carry out a measurement on the patient's respiratory air flow, second means (8) arranged to calculate the respiratory volume on the basis of the performed measurement, and a control unit (7) arranged to determine the amount of water discharged from the patient on the basis of the measured respiratory volume, and an actuator (4) and a nozzle piece (3) that are arranged, according to directions from the control unit (7), to supply an amount of water, dependent on the amount of water discharged from the patient, to the patient's respiratory tract during the next respiratory phase(s).

2. An arrangement according to claim 1, **characterized in that** the actuator (4) comprises a container (12) arranged to receive an amount of water dependent on the amount of water discharged from the patient, and that the aforementioned amount of water is arranged to be supplied to the patient by emptying the container (12) at one go.

3. An arrangement according to claim 2, **characterized in that** the container (12) of the actuator (4) is formed of a cylinder with an associated piston (11).

4. An arrangement according to claim 1, **characterized in that** the arrangement also comprises a component (14) that comprises an artificial nose, a bacterial filter or both.

5. An arrangement according to claim 1, **characterized in that** the duration of the water supply and the timing during the respiration, usually at the beginning of inhalation or exhalation, are arranged to be selected to suit each situation.

6. An arrangement according to claim 5, **characterized in that** with the adjustment of the duration and timing of the water supply, humidity is arranged to be guided in weighted amounts to different parts of the air passage and the lungs (1).

7. An arrangement according to claims 5 or 6, **characterized in that** the flow sensor (6) provides the timing signal for the water supply.

8. An arrangement according to claims 5 or 6, **characterized in that** a ventilator or a respirator (9) provides the timing signal for the water supply.

9. An arrangement according to any one of the preceding claims, **characterized by** means to convert the water supplied to the patient into the form of aerosol.

10. An arrangement according to any one of preceding claims 1 to 8, **characterized by** means (13) to heat the water supplied to the patient into water vapour before it is conducted to the respiratory tract.

11. An arrangement according to claim 9, **characterized in that** the means (13) to heat the water supplied to the patient also heats the inhalation gas.

12. An arrangement according to any one of the preceding claims, **characterized in that** the water supplied to the patient includes a pharmaceutical.

13. An arrangement according to any one of the preceding claims, **characterized in that** the flow sensor (6) measures the single respiratory volume or minute volume measured from the exhalation.

14. An arrangement according to any one of the preceding claims, **characterized in that** the amount of water to be supplied to the patient is proportional to the amount of water discharged from the patient.

15. An arrangement according to any one of the preceding claims, **characterized by** means to supply the water to the patient through spraying.

16. An arrangement according to claim 12, **characterized in that** the pharmaceutical added to the water is water-soluble.

17. An arrangement according to any one of the preceding claims, **characterized in that** the control unit (7) determines the amount of water discharged from the patient on the basis of the measured volume and the assumed or measured temperature.

## Patentansprüche

1. Anordnung zur Behandlung von Atemgas eines intubierten Patienten, welche Anordnung Mittel (2, 9) zur Zuführung von Inhalationsgas zu den Lungen (1) des Patienten umfasst und um das Exhalationsgas von dem Patienten zu leiten, und Befeuchtungsmittel (5), um den Feuchtigkeitsgrad in dem Inhalationsgas zu erhöhen, **dadurch gekennzeichnet, dass** die Anordnung weiter einen Durchflussmesser (6) umfasst, welcher angeordnet ist, um eine Messung des Atemgasluftflusses des Patienten durchzuführen, zweite Mittel (8), angeordnet, um das Atemvolumen auf der Basis der durchgeführten Messung zu berechnen, und eine Steuereinheit (7), welche angeordnet ist, um die von dem Patienten ausgegebene Menge an Wasser auf der Basis des gemessenen Atemvolumens zu bestimmten, und ein Betätigungsmittel (4) und ein Mundstück (3), welche angeordnet sind, um, gemäß Anweisungen von der Steuereinheit (7), dem Patientenatemgastrakt während der (den) nächsten Atemphase(n) eine Menge an Wasser zuzuführen, welche von der Menge an von dem Patienten abgegebenen Wasser abhängig ist.

2. Anordnung nach Anspruch 1, **dadurch gekennzeichnet, dass** das Betätigungsmittel (4) einen Container (12) umfasst, welcher angeordnet ist, um eine Menge an Wasser aufzunehmen, welche von der Menge an von dem Patienten ausgegebenen Wasser abhängig ist, und dass die oben erwähnte Wassermenge angeordnet ist, um dem Patienten durch Leeren des Containers (12) auf einen Zug zugeführt zu werden.

3. Anordnung nach Anspruch 2, **dadurch gekennzeichnet, dass** der Container (12) des Betätigungsmittels (4) aus einem Zylinder mit einem zugehörigen Kolben (11) gebildet ist.

4. Anordnung nach Anspruch 1, **dadurch gekennzeichnet, dass** die Anordnung auch eine Komponente (14) umfasst, die eine künstliche Nase, einen Bakterienfilter oder beides umfasst.

5. Anordnung nach Anspruch 1, **dadurch gekennzeichnet, dass** die Dauer der Wasserzufuhr und das Timing während der Beatmung, üblicherweise an dem Beginn von Inhalation oder Exhalation, eingerichtet sind, um zu jeder Situation zu passen.

6. Anordnung nach Anspruch 5, **dadurch gekennzeichnet, dass** mit der Einstellung der Dauer und des Timings der Wasserzufuhr Feuchtigkeit eingerichtet ist, um in gewichteten Mengen zu verschiedenen Teilen der Luftpassage und der Lungen (1) geführt zu werden.

7. Anordnung nach einem der Ansprüche 5 oder 6, **dadurch gekennzeichnet, dass** der Durchflusssensor (6) das Timingsignal für die Wasserzufuhr bietet.

8. Anordnung nach einem der Ansprüche 5 oder 6, **dadurch gekennzeichnet, dass** ein Belüfter oder ein Beatmungsgerät (9) das Timingsignal für die Wasserzufuhr liefert.

9. Anordnung nach einem der vorhergehenden Ansprüche, **gekennzeichnet durch** Mittel, um das zu dem Patienten zugeführte Wasser in die Form von Aerosol umzuwandeln.

10. Anordnung nach einem der vorhergehenden Ansprüche 1 - 8, **gekennzeichnet durch** Mittel (13), um das zu dem Patienten zugeführte Wasser zu Wasserdampf zu erhitzen, bevor es in den Atemtrakt geleitet wird.

11. Anordnung nach Anspruch 9, **dadurch gekennzeichnet, dass** die Mittel (13), um das zu dem Patienten zugeführte Wasser zu erwärmen auch das Inhalationsgas erwärmen.

12. Anordnung nach einem der vorhergehenden Ansprüche, **dadurch gekennzeichnet, dass** das zu dem Patienten zugeführte Wasser einen pharmazeutischen Stoff enthält.

13. Anordnung nach einem der vorhergehenden Ansprüche, **dadurch gekennzeichnet, dass** der Durchflusssensor (6) das einzelne Atemvolumen oder von der Exhalation gemessene Minutenvolumen misst.

14. Anordnung nach einem der vorhergehenden Ansprüche, **dadurch gekennzeichnet, dass** die Menge an dem Patienten zuzuführendem Wasser zu der Menge an von dem Patienten ausgegebenen Wasser proportional ist.

15. Anordnung nach einem der vorhergehenden Ansprüche, **gekennzeichnet durch** Mittel, um dem Patienten das Wasser **durch** Sprühen zuzuführen.

16. Anordnung nach Anspruch 12, **dadurch gekennzeichnet, dass** der dem Wasser hinzugefügte pharmazeutische Stoff wasserlöslich ist.

17. Anordnung nach einem der vorhergehenden Ansprüche, **dadurch gekennzeichnet, dass** die Steuereinheit (7) die Menge an von dem Patienten abgegebenen Wasser auf der Basis des gemessenen Volumens und der angenommenen oder gemessenen Temperatur bestimmt.

## Revendications

1. Arrangement pour le traitement du gaz respiratoire d'un patient intubé, l'arrangement comprenant des moyens (2, 9) pour délivrer du gaz respiratoire dans les poumons (1) du patient et pour évacuer le gaz expiré par le patient, et des moyens d'humidification (5) pour augmenter le niveau d'humidité du gaz respiratoire, **caractérisé en ce que** l'arrangement comprend encore un débitmètre (6) pour mesurer le débit d'air respiratoire du patient, de deuxièmes moyens (8) pour calculer le volume respiratoire sur la base de la mesure, et une unité de contrôle (7) pour déterminer sur la base du volume respiratoire mesuré la quantité d'eau évacuée du patient, et un vérin (4) et une canule (3) pour délivrer selon les instructions reçues de l'unité de contrôle (7) une quantité d'eau, qui dépend de la quantité d'eau évacuée du patient, à la voie respiratoire du patient pendant le(s) phase(s) respiratoire(s) suivante(s).

2. Arrangement selon la revendication 1, **caractérisé en ce que** le vérin (4) comprend un récipient (12) pour recevoir une quantité d'eau qui dépend de la quantité d'eau évacuée du patient, et **en ce que** ladite quantité d'eau est prévue d'être délivrée au patient en évacuant tout le récipient (12) à la fois.

3. Arrangement selon la revendication 2, **caractérisé en ce que** le récipient (12) du vérin (4) est composé d'un cylindre et d'un piston (11) associé.

4. Arrangement selon la revendication 1, **caractérisé en ce que** l'arrangement comprend encore un composant (14) qui comprend un nez artificiel, un filtre bactérien ou les deux.

5. Arrangement selon la revendication 1, **caractérisé en ce qu'**il est prévu de choisir la durée et le rythme de l'alimentation d'eau pendant la respiration, normalement au début de l'inspiration ou de l'expiration, de sorte qu'ils soient appropriés à chaque situation.

6. Arrangement selon la revendication 5, **caractérisé en ce que** grâce à l'ajustement de la durée et du rythme de l'alimentation d'eau, l'humidité est prévue d'être dirigée en des quantités pondérées à des parties différentes des voies aériennes et des poumons (1).

7. Arrangement selon la revendication 5 ou 6, **caractérisé en ce que** le débitmètre (6) fournit le signal de rythme de l'alimentation d'eau.

8. Arrangement selon la revendication 5 ou 6, **caractérisé en ce qu'**un ventilateur ou un respirateur (9) fournit le signal du rythme de l'alimentation d'eau.

9. Arrangement selon l'une quelconque des revendications précédentes, **caractérisé en ce qu'**il comprend des moyens pour transformer l'eau alimentée au patient en une forme d'aérosol.

10. Arrangement selon l'une quelconque des revendications précédentes 1 à 8, **caractérisé en ce qu'**il comprend des moyens (13) pour chauffer l'eau alimentée au patient et la transformer en vapeur d'eau avant qu'elle soit délivrée aux voies respiratoires.

11. Arrangement selon la revendication 9, **caractérisé en ce que** le moyen (13) pour chauffer l'eau alimentée au patient aussi chauffe le gaz d'inspiration.

12. Arrangement selon l'une quelconque des revendications précédentes, **caractérisé en ce que** l'eau alimentée au patient contient un médicament.

13. Arrangement selon l'une quelconque des revendications précédentes, **caractérisé en ce que** le débitmètre (6) mesure un volume unique ou un volume par minute mesuré de la respiration.

14. Arrangement selon l'une quelconque des revendications précédentes, **caractérisé en ce que** la quantité d'eau à alimenter au patient est proportionnelle à la quantité d'eau évacuée du patient.

15. Arrangement selon l'une quelconque des revendications précédentes, **caractérisé en ce qu'**il comprend des moyens pour alimenter en eau le patient par pulvérisation.

16. Arrangement selon la revendication 12, **caractérisé en ce que** le médicament ajouté dans l'eau est hydrosoluble.

17. Arrangement selon l'une quelconque des revendications précédentes, **caractérisé en ce que** l'unité de contrôle (7) détermine la quantité d'eau évacuée du patient sur la base du volume mesuré et la température présumée ou mesurée.
